# EUROPEAN PATENT SPECIFICATION

(11) **EP 2 830 714 B1**
(45) Date of publication and mention of the grant of the patent: **27.09.2017**
(21) Application number: 13715860.6
(22) Date of filing: 28.03.2013
(51) Int. Cl.: A61Q 5/12, A61K 8/41, A61K 8/34, A61K 8/81

(54) **HAIR CONDITIONING COMPOSITION COMPRISING CATIONIC SURFACTANT AND DEPOSITION POLYMER**
HAARKONDITIONIERUNGSZUSAMMENSETZUNG MIT EINEM KATIONISCHEN TENSID UND EINEM ABSCHEIDUNGSPOLYMER
PRÉPARATION DE CONDITIONNEMENT DES CHEVEUX AVEC UN SYSTÈME TENSIOACTIF CATIONIQUE ET UN POLYMÈRE DE DÊPOT

(30) Priority: 30.03.2012 US 201261617736 P
(43) Date of publication of application: 04.02.2015
(73) Proprietor: The Procter & Gamble Company, Cincinnati, OH 45202 (US)
(72) Inventor: UEHARA, Nobuaki, Singapore 138648 (SG); KRISHAN, Kapilanjan, Kobe Hyogo 658-0032 (JP)
(74) Representative: Sauvaître, Thibault Bruno
(86) International application number: PCT/US2013/034199
(87) International publication number: WO 2013/148905

(56) References cited:
- EP-A2- 0 668 070
- WO-A1-2009/153280
- WO-A1-2010/026097
- WO-A2-2009/158438
- US-A1- 2008 286 218

## Description

### FIELD OF THE INVENTION

The present invention relates to a hair conditioning composition comprising: a cationic surfactant; a high melting point fatty compound; a deposition polymer having specific monomers; and an aqueous carrier; wherein the mole % of the cationic surfactant to a sum of the cationic surfactant and the high melting point fatty compound is from about 20% to about 60%. The composition of the present invention provides improved friction reduction on wet hair, while providing improved deposition of cationic surfactant, fatty compounds, and/or silicone compounds.

### BACKGROUND OF THE INVENTION

A variety of approaches have been developed to condition the hair. A common method of providing conditioning benefit is through the use of conditioning agents such as cationic surfactants, high melting point fatty compounds, silicone compounds, and mixtures thereof. Most of these conditioning agents are known to provide various conditioning benefits.

There have been trials for conditioners to provide improved conditioning benefits. For example, Japanese Patent Application Laid-Open No. 2007-137830 discloses hair cosmetics comprising a cationic surfactant, a fatty alcohol, a silicone, and a polymer containing hydrophilic nonionic monomers and anionic monomers. Japanese Patent Application Laid-Open No. 2007-137830 also describes that such hair cosmetics provide superior conditioning benefits.

However, there is still a need for rinse-off conditioners to provide improved deposition of conditioning agents on the hair, especially on damaged hair. By improved deposition of conditioning agents, such rinse-off conditioners can provide either: improved conditioning benefits from the same amount of the conditioning agents; or conditioning benefits effectively from the reduced amount of the conditioning agents.

There is also a need for rinse-off conditioners to provide improved friction reduction on wet hair.

None of the existing art provides all of the advantages and benefits of the present invention.

### SUMMARY OF THE INVENTION

The present invention is directed to a hair conditioning composition comprising by weight:
(a)from about 0.1% to about 8% of a cationic surfactant;
(b) from about 1% to about 15% of a high melting point fatty compound;
(c) from about 0.05% to about 6% of a deposition polymer which is a copolymer comprising: a vinyl monomer (A) with a carboxyl group in the structure; and a vinyl monomer (B) expressed by the following formula (1):

   CH₂=C(R¹)-CO-X-(Q-O)ᵣ-R² (1)

   wherein: R¹ represents a hydrogen atom or a methyl group; R² represents a hydrogen atom or an alkyl group with from 1 to 5 carbon atoms, which may have a substitution group; Q represents an alkylene group with from 2 to 4 carbon atoms which may also have a substitution group; r represents an integer from 2 to 15; and X represents an oxygen atom or an NH group; and, in the following structure - (Q - O)ᵣ - R², the number of atoms bonded in a straight chain is 70 or less; and wherein the vinyl monomer (A) is contained at a level of from about 10 mass% to about 50 mass%, and the vinyl monomer (B) is contained at level of from about 50 mass% to about 90 mass%; and
(d) an aqueous carrier;
   wherein the mole % of the cationic surfactant to a sum of the cationic surfactant and the high melting point fatty compound is from about 20% to about 60%.

The composition of the present invention provides improved friction reduction on wet hair, while providing improved deposition of cationic surfactant, fatty compounds, and/or silicone compounds.

These and other features, aspects, and advantages of the present invention will become better understood from a reading of the following description, and appended claims.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

### CATIONIC SURFACTANT

The compositions of the present invention comprise a cationic surfactant. The cationic surfactant is included in the composition at a level of from about 0.1% to about 8%, preferably from about 0.2% to about 6%, more preferably from about 0.5% to about 5% by weight of the composition.

In the present invention, the cationic surfactant is included such that the mole % of the cationic surfactant to a sum of the cationic surfactant and the high melting point fatty compound is from about 20% to about 60%, preferably from about 22% to about 50%, more preferably from about 25% to about 35%. If the mole% is too low, the composition provides inferior wet friction. If the mole% is too high, the composition may provide an inferior product texture.

Cationic surfactants useful herein include, for example, mono-alkyl quaternized ammonium salt cationic surfactant having one long alkyl chain of from 12 to 30 carbon atoms, mono-alkyl amine cationic surfactant having one long alkyl chain of from 12 to 30 carbon atoms including mono-alkyl amidoamine cationic surfactant. Mono-alkyl quaternized ammonium salt cationic surfactants are preferred. Additionally, di-alkyl quaternized ammonium salt cationic surfactant having two long alkyl chain of from 12 to 30 carbon atoms may be used together with the above mono-alkyl cationic surfactants.

### MONO-ALKYL QUATERNIZED AMMONIUM SALT CATIONIC SURFACTANT

The compositions of the present invention preferably comprise a mono-alkyl quaternized ammonium salt cationic surfactant. The mono-alkyl quaternized ammonium salt cationic surfactant is included in the composition at a level of from about 0.1% to about 8%, preferably from about 0.2% to about 6%, more preferably from about 0.5% to about 5% by weight of the composition.

In the present invention, the mono-alkyl quaternized ammonium salt cationic surfactant is included such that the mole % of the mono-alkyl quaternized ammonium salt cationic surfactant to a sum of the mono-alkyl quaternized ammonium salt cationic surfactant and the high melting point fatty compound is from about 20% to about 60%, preferably from about 22% to about 50%, more preferably from about 25% to about 35%. If the mole% is too low, the compositions tend to provide increased wet friction. If the mole% is too high, the composition may provide an inferior product texture.

The mono-alkyl quaternized ammonium salt cationic surfactants useful herein are those having one long alkyl chain of preferably from 12 to 30 carbon atoms, more preferably from 16 to 24 carbon atoms, still more preferably from 18 to 22 carbon atoms, even more preferably 22 carbon atoms, in view of conditioning benefits. Such mono-alkyl quaternized ammonium salt cationic surfactants useful herein are, for example, those having the formula (I): 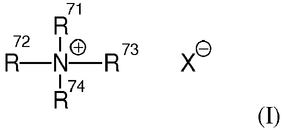 wherein one of R⁷¹, R⁷², R⁷³ and R⁷⁴ is selected from an aliphatic group of from 12 to 30 carbon atoms, more preferably from 16 to 24 carbon atoms, still more preferably from 18 to 22 carbon atoms, even more preferably 22 carbon atoms or an aromatic, alkoxy, polyoxyalkylene, alkylamido, hydroxyalkyl, aryl or alkylaryl group having up to about 30 carbon atoms; the remainder of R⁷¹, R⁷², R⁷³ and R⁷⁴ are independently selected from an aliphatic group of from 1 to about 8 carbon atoms, preferably from 1 to 3 carbon atoms or an aromatic, alkoxy, polyoxyalkylene, alkylamido, hydroxyalkyl, aryl or alkylaryl group having up to about 8 carbon atoms; and X- is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, e.g., those of about 16 carbons, or higher, can be saturated or unsaturated.

Preferably, one of R⁷¹, R⁷², R⁷³ and R⁷⁴ is selected from an alkyl group of from 12 to 30 carbon atoms, more preferably from 16 to 24 carbon atoms, still more preferably from 18 to 22 carbon atoms, even more preferably 22 carbon atoms; and the remainder of R⁷¹, R⁷², R⁷³ and R⁷⁴ are independently selected from CH₃, C₂H₅, C₂H₄OH, CH₂C₆H₅, and mixtures thereof. Such highly preferred cationic surfactants include, for example, behenyl trimethyl ammonium chloride, methyl sulfate or ethyl sulfate.

### MONO-ALKYL AMINE CATIONIC SURFACTANT

The compositions of the present invention may contain a mono-alkyl amine cationic surfactant. The mono-alkyl amine cationic surfactant can be included in the composition at a level of from about 0.1% to about 8%, preferably from about 0.2% to about 6%, more preferably from about 0.5% to about 5% by weight of the composition.

Mono-alkyl amine cationic surfactants useful herein are primary, secondary, and tertiary amines having one long alkyl or alkenyl group of from about 12 to about 30 carbon atoms, preferably from 16 to 24 carbon atoms, more preferably from 18 to 22 alkyl group. Mono-alkyl amines useful herein also include mono-alkyl amidoamines.

Particularly useful are tertiary amidoamines having an alkyl group of from about 12 to about 22 carbon atoms, preferably from about 16 to about 22 carbon atoms. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al.

The above mono-alkyl amine cationic surfactants are preferably used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid. The acid can be used at a molar ratio of the amine to the acid of from about 1 : 0.3 to about 1 : 2, more preferably from about 1 : 0.4 to about 1 : 1.

### DI-ALKYL QUATERNIZED AMMONIUM SALT CATIONIC SURFACTANT

The composition of the present invention may contain a di-alkyl quaternized ammonium salt cationic surfactant. The di-alkyl quaternized ammonium salt cationic surfactant can be included in the composition at a level of from about 0.05% to about 5%, preferably from about 0.1% to about 4%, more preferably from about 0.2% to about 3% by weight of the composition. When included, it is preferred that the weight ratio of the mono-alkyl cationic surfactant to the di-alkyl quaternized ammonium salt cationic surfactant is from about 1:1 to about 5:1, more preferably from about 1.2:1 to about 5:1, still more preferably from about 1.5:1 to about 4:1, in view of stability in rheology and conditioning benefits.

Di-alkyl quaternized ammonium salt cationic surfactants useful herein are those having two long alkyl chains of from 12 to 30 carbon atoms, more preferably from 16 to 24 carbon atoms, still more preferably from 18 to 22 carbon atoms. Such di-alkyl quaternized ammonium salts useful herein are those having the formula (I): 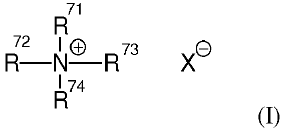 wherein two of R⁷¹, R⁷², R⁷³ and R⁷⁴ are selected from an aliphatic group of from 12 to 30 carbon atoms, preferably from 16 to 24 carbon atoms, more preferably from 18 to 22 carbon atoms or an aromatic, alkoxy, polyoxyalkylene, alkylamido, hydroxyalkyl, aryl or alkylaryl group having up to about 30 carbon atoms; the remainder of R⁷¹, R⁷², R⁷³ and R⁷⁴ are independently selected from an aliphatic group of from 1 to about 8 carbon atoms, preferably from 1 to 3 carbon atoms or an aromatic, alkoxy, polyoxyalkylene, alkylamido, hydroxyalkyl, aryl or alkylaryl group having up to about 8 carbon atoms; and X- is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, e.g., those of about 16 carbons, or higher, can be saturated or unsaturated. Preferably, two of R⁷¹, R⁷², R⁷³ and R⁷⁴ are selected from an alkyl group of from 12 to 30 carbon atoms, preferably from 16 to 24 carbon atoms, more preferably from 18 to 22 carbon atoms; and the remainder of R⁷¹, R⁷², R⁷³ and R⁷⁴ are independently selected from CH₃, C₂H₅, C₂H₄OH, CH₂C₆H₅, and mixtures thereof.

Such preferred di-alkyl cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

### HIGH MELTING POINT FATTY COMPOUND

The composition of the present invention comprises a high melting point fatty compound. The high melting point fatty compound is included in the composition at a level of from about 1% to about 15%, preferably from about 1.5% to about 12%, more preferably from about 2% to about 10% by weight of the composition.

The high melting point fatty compound useful herein have a melting point of 25°C or higher, preferably 40°C or higher, more preferably 45°C or higher, still more preferably 50°C or higher, in view of stability of the emulsion especially the gel matrix. Preferably, such melting point is up to about 90°C, more preferably up to about 80°C, still more preferably up to about 70°C, even more preferably up to about 65°C, in view of easier manufacturing and easier emulsification. In the present invention, the high melting point fatty compound can be used as a single compound or as a blend or mixture of at least two high melting point fatty compounds. When used as such blend or mixture, the above melting point means the melting point of the blend or mixture.

The high melting point fatty compound useful herein is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than the above preferred in the present invention. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are preferably used in the composition of the present invention. The fatty alcohols useful herein are those having from about 14 to about 30 carbon atoms, preferably from about 16 to about 22 carbon atoms. These fatty alcohols are saturated and can be straight or branched chain alcohols.

Preferred fatty alcohols include, for example, cetyl alcohol (having a melting point of about 56°C), stearyl alcohol (having a melting point of about 58-59°C), behenyl alcohol (having a melting point of about 71°C), and mixtures thereof. These compounds are known to have the above melting point. However, they often have lower melting points when supplied, since such supplied products are often mixtures of fatty alcohols having alkyl chain length distribution in which the main alkyl chain is cetyl, stearyl or behenyl group. In the present invention, more preferred fatty alcohols are cetyl alcohol, stearyl alcohol and mixtures thereof.

Commercially available high melting point fatty compounds useful herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan).

### GEL MATRIX

Preferably, in the present invention, a gel matrix is formed by the cationic surfactant system, the high melting point fatty compound, and an aqueous carrier. The gel matrix is suitable for providing various conditioning benefits, such as slippery feel during the application to wet hair and softness and moisturized feel on dry hair.

Preferably, especially when the gel matrix is formed, the total amount of the cationic surfactant and the high melting point fatty compound is from about 4.5%, preferably from about 5.0%, more preferably from about 5.5% by weight of the composition, in view of providing the benefits of the present invention, and to about 15%, preferably to about 14%, more preferably to about 13%, still more preferably to about 10% by weight of the composition, in view of spreadability and product appearance. Furthermore, when the gel matrix is formed, the cationic surfactant and the high melting point fatty compound are contained at a level such that the weight ratio of the cationic surfactant to the high melting point fatty compound is in the range of, preferably from about 1:1 to about 1:10, more preferably from about 1:1.5 to about 1:7, still more preferably from about 1:2 to about 1:6, in view of providing improved wet conditioning benefits.

Preferably, when the gel matrix is formed, the composition of the present invention is substantially free of anionic surfactants, in view of stability of the gel matrix. In the present invention, "the composition being substantially free of anionic surfactants" means that: the composition is free of anionic surfactants; or, if the composition contains anionic surfactants, the level of such anionic surfactants is very low. In the present invention, a total level of such anionic surfactants, if included, preferably 1% or less, more preferably 0.5% or less, still more preferably 0.1% or less by weight of the composition. Most preferably, the total level of such anionic surfactants is 0% by weight of the composition.

### AQUEOUS CARRIER

The composition of the present invention comprises an aqueous carrier. The level and species of the carrier are selected according to the compatibility with other components, and other desired characteristic of the product.

The carrier useful in the present invention includes water and water solutions of lower alkyl alcohols and polyhydric alcohols. The lower alkyl alcohols useful herein are monohydric alcohols having 1 to 6 carbons, more preferably ethanol and isopropanol. The polyhydric alcohols useful herein include propylene glycol, hexylene glycol, glycerin, and propane diol.

Preferably, the aqueous carrier is substantially water. Deionized water is preferably used. Water from natural sources including mineral cations can also be used, depending on the desired characteristic of the product. Generally, the compositions of the present invention comprise from about 20% to about 99%, preferably from about 30% to about 95%, and more preferably from about 80% to about 90% water.

### DEPOSITION POLYMER

The composition of the present invention further comprises a deposition polymer, preferable anionic deposition polymer. The deposition polymer is included at a level by weight of the composition of, from about 0.05% to about 8%, preferably from about 0.1% to about 5%, more preferably from about 0.2% to about 3.5%.

It is preferred that the weight ratio of (i) the deposition polymer to (ii) a sum of the cationic surfactant and high melting point fatty compound is from about 1:1 to about 1:160, more preferably from about 1:2.5 to about 1:120, still more preferably from about 1:3.5 to about 1:80. If the weight ratio of (i) to (ii) is too low, the composition may provide lower deposition of cationic surfactants, high melting point fatty compounds, and/or silicone compounds. If the weight ratio of (i) to (ii) is too high, the composition may influence rheology, and may undesirably decrease rheology of the composition.

The deposition polymer useful herein is a copolymer comprising: a vinyl monomer (A) with a carboxyl group in the structure; and a vinyl monomer (B) expressed by the following formula (1):

CH₂=C(R¹)-CO-X-(Q-O)ᵣ-R² (1)

wherein: R¹ represents a hydrogen atom or a methyl group; R² represents a hydrogen atom or an alkyl group with from 1 to 5 carbon atoms, which may have a substitution group; Q represents an alkylene group with from 2 to 4 carbon atoms which may also have a substitution group; r represents an integer from 2 to 15; and X represents an oxygen atom or an NH group; and, in the following structure - (Q - O)ᵣ - R², the number of atoms bonded in a straight chain is 70 or less; and
wherein the vinyl monomer (A) is contained at a level of from about 10 mass% to about 50 mass%, and the vinyl monomer (B) is contained at level of from about 50 mass% to about 90 mass%.

### Vinyl Monomer (A)

The copolymer of the present invention contains a vinyl monomer (A) having a carboxyl group in the structure. The copolymer may contain one type of the vinyl monomer (A), or may contain two or more types of the vinyl monomer (A). The vinyl monomer (A) is preferably anionic.

This vinyl monomer (A) is contained at a level of from about 10 mass% based on the total mass of the copolymer, preferably from about 15mass%, more preferably 20 mass % or higher, and even more preferably 25 mass% or higher, in view of improved deposition of cationic surfactants, fatty compounds and/or silicones, and to about 50 mass%, preferably 45 mass% or less, and more preferably 40 mass% or less, in view of not-deteriorating smoothness during application and/or the product viscosity.

Non-limited example of the vinyl monomer (A) having a carboxyl group include, for example, unsaturated carboxylic acid monomers having 3 to 22 carbon atoms. The unsaturated carboxylic acid monomer has, preferably 4 or more carbon atoms, and preferably 20 or less carbon atoms, more preferably 18 or less carbon atoms, still more preferably 10 or less carbon atoms, and even more preferably 6 or less carbon atoms. Furthermore, the number of carboxyl groups in the vinyl monomer (A) is preferably from 1 to 4, more preferably from 1 to 3, even more preferably from 1 to 2, and most preferably 1.

In view of improved deposition of cationic surfactants, fatty compounds and/or silicones, the vinyl monomer (A) is preferably an unsaturated carboxylic acid monomer expressed by the following formula (2) or formula (3), more preferably those expressed by the formula (2)

CH₂=C(R³)-CO-(O-(CH₂)ₘ-CO)ₙ-OH (2)

wherein: R³ represents a hydrogen atom or a methyl group, preferably a hydrogen atom; m represents an integer of 1 through 4, preferably 2 to 3; and n represents an integer of 0 through 4, preferably 0 to 2, and most preferably 0

CH₂=C(R⁴)-COO-(CH₂)p-OOC-(CH₂)q-COOH (3)

wherein: R⁴ represents a hydrogen atom or a methyl group, preferably a hydrogen atom; p and q independently represent an integer of 2 through 6, preferably 2 to 3.

Examples of those expressed by the formula (2) include (meth)acrylic acid, crotonic acid, maleic acid, fumaric acid, itaconic acid, angelic acid, tiglic acid, 2-carboxy ethyl acrylate oligomer, and the like. Among them, preferred are acrylic acid and methacrylic acid, and more preferred is acrylic acid. Examples of those expressed by the formula (3) include acryloyloxy ethyl succinate, 2-methacryloyloxy ethyl succinate, and the like.

### Vinyl Monomer (B)

The copolymer contains a vinyl monomer (B). The copolymer may contain one type of the vinyl monomer (B), or may contain two or more types of the vinyl monomer (B). The vinyl monomer (B) is preferably nonionic.

The vinyl monomer (B) is contained at a level of from about 50 mass% based on the total mass of the copolymer in view of improving the feel and the smoothness during application, and to about 90 mass% based on the total mass of the copolymer, preferably to about 85 mass%, more preferably to about 80 mass%, still more preferably 75 mass%, in view of improved deposition of cationic surfactants, fatty compounds and/or silicones.

The Vinyl monomers (B) useful herein are those expressed by formula (4)

CH₂=C(R¹)-CO-X-(Q-O)ᵣ-R² (4)

wherein: R¹ represents a hydrogen atom or a methyl group; R² represents a hydrogen atom or an alkyl group with 1 through 5 carbon atoms, which may have a substitution group; Q represents an alkylene group with 2 through 4 carbon atoms which may also have a substitution group; r represents an integer from 2 through 15; and X represents an oxygen atom or an NH group; and in the structure - (Q - O)ᵣ - R², the number of atoms bonded in a straight chain is 70 or less.

If R² has a substitution group, the substitution group is a substitution group that does not react with other parts of the copolymer. The vinyl monomer (B) is preferably hydrophilic, and therefore R² is preferably a hydrogen atom or an alkyl group with 1 ∼ 3 carbon atoms, and more preferably a hydrogen atom or an alkyl group with 1 or 2 carbon atoms.

X preferably represents an oxygen atom.

Q represents preferably an alkylene group with 2 through 3 carbon atoms which may also have a substitution group, and more preferably an alkylene group with 2 through 3 carbon atoms without any substitution group. If the alkylene group of Q has a substitution group, it is preferred that such substitution group does not react with other parts of the copolymer, more preferably such substitution group has a molecular weight of 50 or less, still more preferably such substitution group has a molecular weight that is smaller than the structural moiety of - (Q - O)r -. Examples of such substitution group include a hydroxyl group, methoxy group, ethoxy group, and the like.

r represents preferably 3 or higher, and preferably 12 or less, in view of improved deposition of cationic surfactants, fatty compounds and/or silicones, and/or in view of smoothness during application.

As described above, in the structure - (Q - O)r - R², the number of atoms that are bonded by the straight chain is 70 or less. For example, if Q represents an n-butylene group, r = 15, and R² represents an n-pentyl group, the number of atoms that are bonded in the straight chain of the structure - (Q - O)r - R² is calculated as 80, which therefore is outside of the scope. The number of atoms bonded in the straight chain in the structure - (Q - O)r - R² is preferably 60 or less, more preferably 40 or less, even more preferably 28 or less, and particularly preferably 20 or less, in view of improved deposition of cationic surfactants, fatty compounds and/or silicones, and/or in view of smoothness during application.

Examples of the vinyl monomer (B) include, methoxy polyethylene glycol (meth)acrylate (where the number of repetitions of polyethylene glycol (r in formula (4)) is between 2 ∼ 15), polyethylene glycol (meth)acrylate (where the number of repetitions of polyethylene glycol (r in formula (4)) is between 2 ∼ 15), methoxy polyethylene glycol / polypropylene glycol (meth)acrylate (where the number of repetitions of polyethylene glycol / polypropylene glycol (r in formula (4)) is between 2 ∼ 15), polyethylene glycol / polypropylene glycol (meth)acrylate (where the number of repetitions of polyethylene glycol / polypropylene glycol (r in formula (4)) is between 2 ∼ 15), methoxy polyethylene glycol / polybutylene glycol (meth)acrylate (where the number of repetitions of polyethylene glycol / polybutylene glycol (r in formula (4)) is between 2 ∼ 15), polyethylene glycol / polybutylene glycol (meth)acrylate (where the number of repetitions of polyethylene glycol / polybutylene glycol (r in formula (4)) is between 2 ∼ 15), methoxy polyethylene glycol (meth)acrylamide (where the number of repetitions of polyethylene glycol (r in formula (4)) is between 2 ∼ 15), and polyethylene glycol (meth)acrylamide (where the number of repetitions of polyethylene glycol (r in formula (4)) is between 2 ∼ 15); preferably methoxy polyethylene glycol (meth)acrylate (where the number of repetitions of polyethylene glycol (r in formula (4)) is between 3 ∼ 12), polyethylene glycol (meth)acrylate (where the number of repetitions of polyethylene glycol (r in formula (4)) is between 3 ∼ 12), methoxy polyethylene glycol / polypropylene glycol (meth)acrylate (where the number of repetitions of polyethylene glycol / polypropylene glycol (r in formula (4)) is between 3 ∼ 12), polyethylene glycol / polypropylene glycol (meth)acrylate (where the number of repetitions of polyethylene glycol / polypropylene glycol (r in formula (4)) is between 3 ∼ 12), methoxy polyethylene glycol / polybutylene glycol (meth)acrylate (where the number of repetitions of polyethylene glycol / polybutylene glycol (r in formula (4)) is between 3 ∼ 12), polyethylene glycol / polybutylene glycol (meth)acrylate (where the number of repetitions of polyethylene glycol / polybutylene glycol (r in formula (4)) is between 3 ∼ 12); more preferably methoxy polyethylene glycol (meth)acrylate (where the number of repetitions of polyethylene glycol (r in formula (4)) is between 3 ∼ 12), and polyethylene glycol (meth)acrylate (where the number of repetitions of polyethylene glycol (r in formula (4)) is between 3 ∼ 12).

### Vinyl Monomer (C)

In addition to the vinyl monomers (A) and (B), the copolymer may further contain a vinyl monomer (C) having an alkyl group with 12 ∼ 22 carbon atoms, in view of providing conditioning effect such as smoothness during application. When included, the amount of the vinyl monomer (C) is preferably 40 mass% or less, more preferably 30 mass% or less, even more preferably 25 mass% or less, and still more preferably 20 mass% or less based on the total mass of the copolymer, in view of improved deposition of cationic surfactants, fatty compounds and/or silicones, and/or in view of smoothness during application.

Preferably, the vinyl monomer (C) is a (meth)acrylate monomer having an alkyl group with 12 ∼ 22 carbon atoms, in view of smoothness during application. Furthermore, vinyl monomers with branched alkyl groups are particularly preferred.

Examples of the (meth)acrylate monomer having an alkyl group with 12 ∼ 22 carbon atoms include myristyl (meth)acrylate, isostearyl (meth)acrylate, stearyl (meth)acrylate, behenyl (meth)acrylate, cetyl (meth)acrylate, lauryl (meth)acrylate, synthetic lauryl (meth)acrylate, (however "synthetic lauryl (meth)acrylate" refers to an alkyl (meth)acrylate having alkyl groups with 12 carbon atoms and alkyl groups with 13 carbon atoms), and the like. Of these, (meth)acrylate monomers having an alkyl group with 12 ∼ 20 carbon atoms are preferable, and (meth)acrylate monomers having an alkyl group with 16 ∼ 18 carbon atoms are more preferable.

The copolymer may contain one type of the vinyl monomer (C), or may contain two or more types of the vinyl monomer (C).

### Other Monomers

In addition to the aforementioned vinyl monomers (A), (B), and (C), the copolymer may also contain other vinyl monomers, to the extent not to deteriorate the effect of the copolymer. Examples of other vinyl monomers include nonionic monomers, amphoteric monomers, semipolar monomers, cationic monomers, as well as monomers containing a polysiloxane group., preferably nonionic monomers with or without polysiloxane group These other monomers are different from any of the aforementioned vinyl monomers (A), (B), and (C).

Normally the amount of such other monomers, if included, is 40 mass% or less of the total mass of the copolymer, preferably 30 mass% or less, more preferably 20 mass% or less, and even more preferably 10 mass% or less.

In view of improved deposition of cationic surfactants, fatty compounds, and/or silicones, the amount of cationic functional groups in the copolymer is preferably low, and for example cationic functional groups preferably account for 10 mole % or less of all functional groups in the copolymer. More preferably, the copolymer is free of cationic functional groups.

Examples of nonionic monomers include esters of (meth)acrylic acid and alcohols with 1 ∼ 22 carbon atoms, amides of (meth)acrylic acid and alkyl amines with 1 ∼ 22 carbon atoms, monoesters of (meth)acrylic acid and ethylene glycol, 1,3-propylene glycol or the like, as well as esters where the hydroxyl group of the monoester has been etherified by methanol, ethanol or the like, (meth)acryloyl morpholine and the like.

Examples of amphoteric monomers include (meth)acryl esters having a betaine group, (meth)acrylamide having a betaine group and the like.

Examples of semipolar monomers include (meth)acrylate esters having an amine oxide group, (meth)acrylamides having an amine oxide group, and the like.

Examples of cationic monomers include (meth)acrylate esters having a quaternary ammonium group, (meth)acrylamides having a quaternary ammonium group and the like.

The monomer containing a polysiloxane group is a monomer having a polysiloxane structure and also having a structure that can bond by covalent bond to the copolymer. These component units have high affinity towards silicone oil that is normally used in conjunction in cosmetic material compositions, and are thought to act by bonding the silicone oil to the other component units in the copolymer and thus increasing the adsorption force of silicone oil to the skin and hair, particularly damaged hair.

The polysiloxane structure is a structure where two or more repeating structural units expressed by the following formula (4) are linked.

-(SiR⁵R⁶-O)- (4)

In formula (4), R⁵ and R⁶ independently represent an alkyl group with 1 to 3 carbon atoms or a phenyl group.

The structure that can link via covalent bond to the copolymer can be a structure that has a vinyl structure such as a (meth)acrylate ester, or (meth)acrylamide and that can copolymerize with another monomer, a structure that has a functional group such as a thiol, that can link to the copolymer by chain transfer during polymerization, or a structure that has an isocyanate group, carboxylic acid group, hydroxyl group, amino group, or the like, and that can react and link to the functional groups on the copolymer, but there is no restriction to these structures.

A plurality of these linkable structures can be present in one monomer containing a polysiloxane group. In the copolymer, the polysiloxane structure can link by a graft structure to the main chain, or conversely the polysiloxane structure can be the main chain with the other structure link by a graft structure, and in addition the polysiloxane structure and the other structure can be linked in a straight chain condition by a block structure.

The monomer containing a polysiloxane group is preferably expressed by the following formula (5).

CH₂=C(R⁷)-Z-(SiR⁸R⁹-O)ₛ-R¹⁰ (5)

In the formula, R⁷ represents a hydrogen atom or a methyl group, R⁸ and R⁹ independently represent an alkyl group with 1 to 3 carbon atoms or a phenyl group, R¹⁰ represents an alkyl group with 1 to 8 carbon atoms, Z represents a bivalent linking group or a direct bond, and s represents an integer between 2 to 200.

More preferably, s is 3 or higher, and even more preferably, s is 5 or higher, in view of increased affinity to silicone oil, and preferably s is 50 or less, in view of enhanced copolymerization with the other monomers.

Z represents a bivalent linking group or a direct bond, but a linking group containing one or a combination of two or more of the structures suggested below is preferable. The numbers that are combined is not particularly restricted, but normally is 5 or less. Furthermore, the direction of the following structures are arbitrary (the polysiloxane group side can be on either end). Note, in the following, R represents an alkylene group with 1 to 6 carbon atoms or a phenylene group.
-COO-R-
-CONH-R-
-O-R-
-R-

The monomer expressed by the aforementioned formula (5), include, for example, α-(vinyl phenyl) polydimethyl siloxane, α-(vinyl benzyloxy propyl) polydimethyl siloxane, α-(vinyl benzyl) polymethyl phenyl siloxane, α-(methacryloyl oxypropyl) polydimethyl siloxane, α-(methacryloyloxy propyl) polymethyl phenyl siloxane, α-(methacryloyl amino propyl) polydimethyl siloxane and the like. The monomer containing a polysiloxane group can be a single type, or can be two or more types used in combination.

In order to adjust the molecular weight and the viscosity of the copolymer, a cross-linking agent such as a polyfunctional acrylate or the like can be introduced to the copolymer. However, in this invention, it is preferred that a cross-linking agent is not included in the copolymer.

### Structure Analysis

The amount of the vinyl monomers (A), (B), and (C) as well as other monomers in the copolymer can be measured using IR absorption or Raman scattering by the carbonyl groups, amide bonds, polysiloxane structures, various types of functional groups, carbon backbone and the like, by ¹H-NMR of methyl groups in the polydimethyl siloxane, amide bond sites, and methyl groups and methylene groups adjacent thereto, as well as various types of NMR represented by ¹³C-NMR and the like.

### Weighted Average Molecular Weight

The weighted average molecular weight of the copolymer is preferably 3,000 or higher, more preferably 5,000 or higher, and even more preferably 10,000 or higher, in view of providing conditioning effect via foaming a complex with cationic surfactant, and preferably to about 2,000,000, more preferably 1,000,000 or less, still more preferably 500,000 or less, even more preferably 100,000 or less, and most preferably 50,000 or less, in view of feeling after drying.

The weighted average molecular weight of the copolymer can be measured by gel permeation chromatography (GPC). The development solvent that is used in gel permeation chromatography is not particularly restricted so long as being a normally used solvent, but for example, the measurement can be performed using a solvent blend of water / methanol / acetic acid / sodium acetate.

### Viscosity

The copolymer preferably has a viscosity for a 20 mass% ethanol solution at 25°C of 5 mPa•s or higher and 20,000 mPa•s or less. The viscosity is more preferably 10 mPa•s or higher, even more preferably 15 mPa•s or higher, but on the other hand is more preferably 10,000 mPa•s or less, and even more preferably 5,000 mPa•s or less. The viscosity of the copolymer is preferably 5 mPa•s or higher and 20,000 mPa•s or less, from the perspective of handling. The viscosity can be measured using a B-type viscometer.

Similar to the weighted average molecular weight, the viscosity of the copolymer can be adjusted by controlling the degree of polymerization of the copolymer, and can be controlled by increasing or decreasing the amount of a cross-linking agent such as a polyfunctional acrylate or the like that is added.

### SILICONE COMPOUND

The compositions of the present invention comprise a silicone compound. The silicone compounds are included at levels by weight of the composition of from about 0.05% to about 15%, preferably from about 0.1% to about 10%, more preferably from about 0.1% to about 8%.

Preferably, the silicone compounds have an average particle size of from about 1microns to about 50 microns, in the composition.

The silicone compounds useful herein, as a single compound, as a blend or mixture of at least two silicone compounds, or as a blend or mixture of at least one silicone compound and at least one solvent, have a viscosity of preferably from about 1,000 to about 2,000,000mPa•s at 25°C.

The viscosity can be measured by means of a glass capillary viscometer as set forth in Dow Corning Corporate Test Method CTM0004, July 20, 1970. Suitable silicone fluids include polyalkyl siloxanes, polyaryl siloxanes, polyalkylaryl siloxanes, polyether siloxane copolymers, amino substituted silicones, quaternized silicones, and mixtures thereof. Other nonvolatile silicone compounds having conditioning properties can also be used.

Silicone compounds useful herein also include amino substituted materials. Preferred aminosilicones include, for example, those which conform to the general formula (I):

(R₁)ₐG₃₋ₐ-Si-(-OSiG₂)ₙ-(-OSiG_{b}(R₁)_{2-b})ₘ-O-SiG₃₋ₐ(R1)ₐ

wherein G is hydrogen, phenyl, hydroxy, or C₁-C₈ alkyl, preferably methyl; a is 0 or an integer having a value from 1 to 3, preferably 1; b is 0, 1 or 2, preferably 1; n is a number from 0 to 1,999; m is an integer from 0 to 1,999; the sum of n and m is a number from 1 to 2,000; a and m are not both 0; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A⁻; -N(R₂)CH₂-CH₂-NR₂H₂A ; wherein R₂ is hydrogen, phenyl, benzyl, or a saturated hydrocarbon radical, preferably an alkyl radical from about C₁ to about C₂₀; A is a halide ion.

Highly preferred amino silicones are those corresponding to formula (I) wherein m=0, a=1, q=3, G=methyl, n is preferably from about 1500 to about 1700, more preferably about 1600; and L is -N(CH₃)₂ or -NH₂, more preferably -NH₂. Another highly preferred amino silicones are those corresponding to formula (I) wherein m=0, a=1, q=3, G=methyl, n is preferably from about 400 to about 600, more preferably about 500; and L is -N(CH₃)₂ or -NH₂, more preferably -NH₂. Such highly preferred amino silicones can be called as terminal aminosilicones, as one or both ends of the silicone chain are terminated by nitrogen containing group.

The above aminosilicones, when incorporated into the composition, can be mixed with solvent having a lower viscosity. Such solvents include, for example, polar or non-polar, volatile or non-volatile oils. Such oils include, for example, silicone oils, hydrocarbons, and esters. Among such a variety of solvents, preferred are those selected from the group consisting of non-polar, volatile hydrocarbons, volatile cyclic silicones, non-volatile linear silicones, and mixtures thereof. The non-volatile linear silicones useful herein are those having a viscosity of from about 1 to about 20,000 centistokes, preferably from about 20 to about 10,000 centistokes at 25°C. Among the preferred solvents, highly preferred are non-polar, volatile hydrocarbons, especially non-polar, volatile isoparaffins, in view of reducing the viscosity of the aminosilicones and providing improved hair conditioning benefits such as reduced friction on dry hair. Such mixtures have a viscosity of preferably from about 1,000mPa•s to about 100,000mPa•s, more preferably from about 5,000mPa•s to about 50,000mPa•s.

Other suitable alkylamino substituted silicone compounds include those having alkylamino substitutions as pendant groups of a silicone backbone. Highly preferred are those known as "amodimethicone". Commercially available amodimethicones useful herein include, for example, BY16-872 available from Dow Corning.

Silicone compounds useful herein also include polyalkyl siloxanes such as polydimethylsiloxane, polydiethylsiloxane, and polymethylphenylsiloxane. Polydimethylsiloxane, which is also known as dimethicone, is especially preferred. These silicone compounds are available, for example, from the General Electric Company in their Viscasil^{®} and TSF 451 series, and from Dow Corning in their Dow Corning SH200 series.

The above polyalkylsiloxanes are available, for example, as a mixture with silicone compounds having a lower viscosity. Such mixtures have a viscosity of preferably from about 1,000mPa•s to about 100,000mPa•s, more preferably from about 5,000mPa•s to about 50,000mPa•s. Such mixtures preferably comprise: (i) a first silicone having a viscosity of from about 100,000mPa•s to about 30,000,000mPa•s at 25°C, preferably from about 100,000mPa•s to about 20,000,000mPa•s; and (ii) a second silicone having a viscosity of from about 5mPa•s to about 10,000mPa•s at 25°C, preferably from about 5mPa·s to about 5,000mPa•s. Such mixtures useful herein include, for example, a blend of dimethicone having a viscosity of 18,000,000mPa•s and dimethicone having a viscosity of 200mPa•s available from GE Toshiba, and a blend of dimethicone having a viscosity of 18,000,000mPa•s and cyclopentasiloxane available from GE Toshiba.

The silicone compounds useful herein also include a silicone gum. The term "silicone gum", as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. The "silicone gums" will typically have a mass molecular weight in excess of about 200,000, generally between about 200,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. The silicone gums are available, for example, as a mixture with silicone compounds having a lower viscosity. Such mixtures useful herein include, for example, Gum/Cyclomethicone blend available from Shin-Etsu.

The silicone compounds may further be incorporated in the present composition in the form of an emulsion, wherein the emulsion is made my mechanical mixing, or in the stage of synthesis through emulsion polymerization, with or without the aid of a surfactant selected from anionic surfactants, nonionic surfactants, cationic surfactants, and mixtures thereof.

### ADDITIONAL COMPONENTS

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present compositions. These include: other conditioning agents such as hydrolysed collagen with tradename Peptein 2000 available from Hormel, vitamin E with tradename Emix-d available from Eisai, panthenol available from Roche, panthenyl ethyl ether available from Roche, hydrolysed keratin, proteins, plant extracts, and nutrients; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; coloring agents, such as any of the FD&C or D&C dyes; perfumes; ultraviolet and infrared screening and absorbing agents such as benzophenones; and antidandruff agents such as zinc pyrithione.

### PRODUCT FORMS and METHOD OF USE

The compositions of the present invention can be in the form of rinse-off products or leave-on products, and can be formulated in a wide variety of product forms, including but not limited to creams, gels, emulsions, mousses and sprays. The composition of the present invention is especially suitable for hair conditioners especially rinse-off hair conditioners.

The composition of the present invention is preferably used for a method of conditioning hair, the method comprising following steps:
(i) after shampooing hair, applying to the hair an effective amount of the conditioning composition for conditioning the hair; and
(ii) then rinsing the hair.

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention. Where applicable, ingredients are identified by chemical or CTFA name, or otherwise defined below.

**Compositions (wt%)**

| | Components | Ex.1 | Ex. 2 | Ex. 3 | CEx i | CEx ii |
|---|---|---|---|---|---|---|
| Group O | Isopropyl alcohol | 0.56 | 0.56 | 0.56 | 0.36 | 0.36 |
| | Behentrimonium methosulfate | 2.26 | 2.26 | 2.26 | 1.42 | 1.42 |
| | Cetyl alcohol | 1.01 | 1.01 | 1.01 | 1.15 | 1.15 |
| | Stearyl alcohol | 2.52 | 2.52 | 2.52 | 2.87 | 2.87 |
| | Benzyl alcohol | 0.4 | 0.4 | 0.4 | 0.4 | 0.4 |
| Group W | Disodium EDTA | 0.13 | 0.13 | 0.13 | 0.13 | 0.13 |
| | Water-soluble preservatives | 0.03 | 0.03 | 0.03 | 0.03 | 0.03 |
| | Deionized Water | q.s. to 100% | | | | |
| Others | Silicone compound * 1 | - | 0.2 | - | - | 0.2 |
| | Deposition polymer-1 *2 | 0.5 | 0.5 | - | 0.5 | 0.5 |
| | Deposition polymer-2 *3 | - | - | 0.5 | - | - |
| Mole % of cationic surfactants to a sum of cationic surfactants (above #2∼5) and high melting point fatty compounds (above #6 and 7) | | About 27% | About 27% | About 27% | About 17% | About 17% |
| Friction reduction on wet hair | | A | S+ | - | C | C- |

### Definitions of Components

*1 Silicone compound: Available from Momentive having a viscosity 10,000mPa•s, and having following formula (I):

   (R₁)ₐG₃₋ₐ-Si-(-OSiG₂)ₙ-(-OSiG_{b}(R₁)_{2-b})ₘ-O-SiG₃₋ₐ(R₁)ₐ (I)

   wherein G is methyl; a is an integer of 1; b is 0, 1 or 2, preferably 1; n is a number from 400 to about 600; m is an integer of 0; R₁ is a monovalent radical conforming to the general formula CqH_{2q}L, wherein q is an integer of 3 and L is -NH₂
*2 Deposition polymer-1: Copolymer of 30wt%of acrylic acid monomer and 70wt% of methoxyPEG-4methacrylate monomer, having a molecular weight of about 24,000.
*3 Deposition polymer-2: Copolymer of 50wt%of acrylic acid monomer and 50wt% of methoxyPEG-4methacrylate monomer, having a molecular weight of about 24,000.

### Method of Preparation

The above hair conditioning compositions of "Ex. 1" through "Ex. 3" and "CEx. i" through "CEx. ii" were prepared by the following method:
Group O components are mixed and heated to from about 66°C to about 85°C to form an oil phase. Separately, Group Wcomponents are mixed and heated to from about 20°C to about 48°C to form an aqueous phase. In Becomix® direct injection rotor-stator homogenizer, the oil phase is injected and it takes 0.2 second or less for the oils phase to reach to a high shear field having an energy density of from 1.0x10⁵ J/m³ to 1.0x10⁷ J/m³ where the aqueous phase is already present. A gel matrix is formed. Other components are added to the gel matrix with agitation. Then the composition is cooled down to room temperature.

### Properties and Conditioning benefits

For some of the above compositions, properties and conditioning benefits are evaluated by the following methods. Results of the evaluation are also shown above.

The embodiments disclosed and represented by "Ex. 1" through "Ex. 3" are hair conditioning compositions of the present invention which are particularly useful for rinse-off use. Such embodiments have many advantages. For example, they provide improved deposition of silicone compound on damaged hair.

Such advantages can be understood by the comparison between the examples of the present invention and comparative examples "CEx. i" through "CEx. ii". For example, improved friction reduction on wet hair was observed in "Ex. 1" and "Ex. 2" of the present invention, compared to comparative examples "CEx. i" and "CEx. ii" which have a lower mole % of the cationic surfactant to a sum of the cationic surfactant and fatty alcohols. Furthermore, by comparison among these four examples, it is observed that addition of silicone into the composition of the present invention provides further improved wet friction reduction, while addition of silicone into the comparative composition having a lower mole% does not provide such synergetic effect.

### Friction reduction on wet hair

Friction force on wet hair is measured by an instrument named Texture Analyzer (TA XT Plus, Texture Technologies, Scarsdale, NY, USA). 1g of the composition is applied to 10g of hair sample. After spreading the composition on the hair sample, rinsing it with warm water for about 1.5minute. During the rinsing, combing the hair sample four times by a polyurethane pad, and friction force (g) between the hair sample and the polyurethane pad is measured by the above instrument each time. An average of four friction forces from four time combing is obtained and evaluated as follows:
S+:Above 40% (excluding 40%) reduction of Friction force, compared to Control
S: Above 30% (excluding 30%) to 40% reduction of Friction force, compared to Control
A: Above 20% (excluding 20%) to 30% reduction of Friction force, compared to Control
B: Above 10% (excluding 10%) to 20% reduction of Friction force, compared to Control
C+: Up to 10% (including 10%) reduction of Friction force, compared to Control
C: Control
C-: Up to 10% (including 10%) increase of Friction force, compared to Control

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A hair conditioning composition comprising by weight:
(a) from about 0.1% to about 8% of a cationic surfactant;
(b) from about 1% to about 15% of a high melting point fatty compound;
(c) from about 0.05% to about 6% of a deposition polymer which is a copolymer comprising: a vinyl monomer (A) with a carboxyl group in the structure; and a vinyl monomer (B) expressed by the following formula (1):
CH₂=C(R¹)-CO-X-(Q-O)ᵣ-R² (1)
wherein: R¹ represents a hydrogen atom or a methyl group; R² represents a hydrogen atom or an alkyl group with from 1 to 5 carbon atoms, which may have a substitution group; Q represents an alkylene group with from 2 to 4 carbon atoms which may also have a substitution group; r represents an integer from 2 to 15; and X represents an oxygen atom or an NH group; and, in the following structure - (Q - O)ᵣ - R², the number of atoms bonded in a straight chain is 70 or less; and wherein the vinyl monomer (A) is contained at a level of from about 10 mass% to about 50 mass%, and the vinyl monomer (B) is contained at level of from about 50 mass% to about 90 mass%; and
(d) an aqueous carrier;
wherein the mole % of the cationic surfactant to a sum of the cationic surfactant and the high melting point fatty compound is from about 20% to about 60%.

2. The composition of Claim 1 wherein, in the formula (1), r represents from about 3 to about 12.

3. The composition of Claim 1 wherein, in the formula (1), X represents an oxygen atom.

4. The composition of Claim 1 wherein the vinyl monomer (A) is expressed by the following formula (2) or the following formula (3):
CH₂=C(R³)-CO-(O-(CH₂)ₘ-CO)ₙ-OH (2)
wherein R³ represents a hydrogen atom or a methyl group, m represents an integer of 1 through 4, and n represents an integer of 0 through 4;
CH₂=C(R⁴)-COO-(CH₂)ₚ-OOC-(CH₂)_{q}-COOH (3)
wherein R⁴ represents a hydrogen atom or a methyl group, p and q independently represent an integer of 2 through 6.

5. The composition of Claim 1 wherein the deposition polymer has a weighted average molecular weight of from about 3,000 to about 2,000,000.

6. The composition of Claim 1 wherein the deposition polymer is anionic.

7. The composition of Claim 1 wherein the cationic surfactant is a mono-alkyl quaternized ammonium salt cationic surfactant having one long alkyl or alkenyl group of from about 12 to about 30 carbon atoms, or its combination with di-alkyl quaternized ammonium salt cationic surfactant having two long alkyl chain of from 12 to 30 carbon atoms.

8. The composition of Claim 1 wherein the cationic surfactant is a mono-alkyl quaternized ammonium salt cationic surfactant having one long alkyl or alkenyl group of 22 carbon atoms, or its combination with di-alkyl quaternized ammonium salt cationic surfactant having two long alkyl chain of from 12 to 30 carbon atoms.

9. The composition of Claim 1 wherein the mole % of the cationic surfactant to a sum of the cationic surfactant and the high melting point fatty compound is from about 22% to about 50%.

10. The composition of Claim 1 wherein the mole % of the cationic surfactant to a sum of the cationic surfactant and the high melting point fatty compound is from about 25% to about 35%.

## Patentansprüche

1. Haarkonditionierungszusammensetzung, umfassend, in Gewichtsprozent:
(a) zu etwa 0,1 % bis etwa 8 % ein kationisches Tensid;
(b) zu etwa 1 % bis etwa 15 % eine Fettverbindung mit hohem Schmelzpunkt;
(c) zu etwa 0,05 % bis etwa 6 % ein Abscheidungspolymer, das ein Copolymer ist, welches umfasst: ein Vinylmonomer (A) mit einer Carboxylgruppe in der Struktur; und ein Vinylmonomer (B), ausgedrückt durch die folgende Formel (1):
CH₂=C(R¹)-CO-X-(Q-O)ᵣ-R² (1)
wobei: R¹ für ein Wasserstoffatom oder eine Methylgruppe steht; R² für ein Wasserstoffatom oder eine Alkylgruppe mit 1 bis 5 Kohlenstoffatomen steht, die eine Substitutionsgruppe aufweisen können; Q für eine Alkylengruppe mit 2 bis 4 Kohlenstoffatomen steht, die ebenfalls eine Substitutionsgruppe aufweisen können; r für eine ganze Zahl von 2 bis 15 steht; und X für ein Sauerstoffatom oder eine NH-Gruppe steht; und, in der folgenden Struktur - (Q - O)ᵣ - R², die Anzahl der in einer geraden Kette gebundenen Atome 70 oder weniger beträgt; und wobei das Vinylmonomer (A) in einer Konzentration von etwa 10 Massen-% bis etwa 50 Massen-% enthalten ist, und das Vinylmonomer (B) in einer Konzentration von etwa 50 Massen-% bis etwa 90 Massen-% enthalten ist; und
(d) einen wässrigen Träger;
wobei die Mol-% des kationischen Tensids zu einer Summe des kationischen Tensids und der Fettverbindung mit hohem Schmelzpunkt etwa 20 % bis etwa 60 % betragen.

2. Zusammensetzung nach Anspruch 1, wobei in der Formel (1) r für etwa 3 bis etwa 12 steht.

3. Zusammensetzung nach Anspruch 1, wobei in der Formel (1) X für ein Sauerstoffatom steht.

4. Zusammensetzung nach Anspruch 1, wobei das Vinylmonomer (A) durch die folgende Formel (2) oder die folgende Formel (3) ausgedrückt wird:
CH₂=C(R³)-CO-(O-(CH₂)ₘ,-CO)ₙ-OH (2)
wobei R³ für ein Wasserstoffatom oder eine Methylgruppe steht, m für eine ganze Zahl von 1 bis 4 steht, und n für eine ganze Zahl von 0 bis 4 steht;
CH₂=C(R⁴)-COO-(CH₂)ₚ-OOC-(CH₂)_{q}-COOH (3)
wobei R⁴ für ein Wasserstoffatom oder eine Methylgruppe steht, p und q unabhängig für eine ganze Zahl von 2 bis 6 stehen.

5. Zusammensetzung nach Anspruch 1, wobei das Abscheidungspolymer ein durchschnittliches Molekulargewicht (Gewichtsmittel) von etwa 3.000 bis etwa 2.000.000 hat.

6. Zusammensetzung nach Anspruch 1, wobei das Abscheidungspolymer anionisch ist.

7. Zusammensetzung nach Anspruch 1, wobei das kationische Tensid ein kationisches Tensid auf Basis von quartärem Monoalkyl-Ammoniumsalz mit einer langen Alkyl- oder Alkylengruppe von etwa 12 bis etwa 30 Kohlenstoffatomen oder dessen Kombination mit einem kationischen Tensid auf Basis von quartärem Dialkyl-Ammoniumsalz mit zwei langen Alkylketten von 12 bis 30 Kohlenstoffatomen ist.

8. Zusammensetzung nach Anspruch 1, wobei das kationische Tensid ein kationisches Tensid auf Basis von quartärem Monoalkyl-Ammoniumsalz mit einer langen Alkyl- oder Alkylengruppe von 22 Kohlenstoffatomen oder dessen Kombination mit einem kationischen Tensid auf Basis von quartärem Dialkyl-Ammoniumsalz mit zwei langen Alkylketten von 12 bis 30 Kohlenstoffatomen ist.

9. Zusammensetzung nach Anspruch 1, wobei die Mol-% des kationischen Tensids zu einer Summe des kationischen Tensids und der Fettverbindung mit hohem Schmelzpunkt etwa 22 % bis etwa 50 % betragen.

10. Zusammensetzung nach Anspruch 1, wobei die Mol-% des kationischen Tensids zu einer Summe des kationischen Tensids und der Fettverbindung mit hohem Schmelzpunkt etwa 25 % bis etwa 35 % betragen.

## Revendications

1. Composition de conditionnement des cheveux comprenant en poids :
(a) d'environ 0,1 % à environ 8 % d'un agent tensioactif cationique ;
(b) d'environ 1 % à environ 15 % d'un composé gras à point de fusion élevé ;
(c) d'environ 0,05 % à environ 6 % d'un polymère de dépôt qui est un copolymère comprenant : un monomère vinylique (A) avec un groupe carboxyle dans la structure ; et un monomère vinylique (B) exprimé par la formule (1) suivante :
CH₂=C(R¹)-CO-X-(Q-O)ᵣ-R² (1)
dans laquelle : R¹ représente un atome d'hydrogène ou un groupe méthyle ; R² représente un atome d'hydrogène ou un groupe alkyle avec de 1 à 5 atomes de carbone, qui peuvent avoir un groupe de substitution ; Q représente un groupe alkylène avec de 2 à 4 atomes de carbone qui peuvent également avoir un groupe de substitution ; r représente un nombre entier allant de 2 à 15 ; et X représente un atome d'oxygène ou un groupe NH ; et, dans la structure suivante - (Q - O)ᵣ - R², le nombre d'atomes liés dans une chaîne linéaire est 70 ou moins ; et dans laquelle le monomère vinylique (A) est contenu à un taux allant d'environ 10 % en masse à environ 50 % en masse, et le monomère vinylique (B) est contenu à un taux allant d'environ 50 % en masse à environ 90 % en masse ; et
(d) un véhicule aqueux ;
dans laquelle le % molaire de l'agent tensioactif cationique par rapport à une somme de l'agent tensioactif cationique et du composé gras à point de fusion élevé va d'environ 20 % à environ 60 %.

2. Composition selon la revendication 1, dans laquelle, dans la formule (1), r représente d'environ 3 à environ 12.

3. Composition selon la revendication 1, dans laquelle, dans la formule (1), X représente un atome d'oxygène.

4. Composition selon la revendication 1, dans laquelle le monomère vinylique (A) est exprimé par la formule (2) suivante ou la formule (3) suivante :
CH₂=C(R³)-CO-(O-(CH₂)ₘ,-CO)ₙ- 2)
dans laquelle R³ représente un atome d'hydrogène ou un groupe méthyle, m représente un nombre entier de 1 à 4, et n représente un nombre entier de 0 à 4 ;
CH₂=C(R⁴)-COO-(CH₂)ₚ-OOC-(CH₂)_{q}-COOH (3)
dans laquelle R⁴ représente un atome d'hydrogène ou un groupe méthyle, p et q représentent indépendamment un nombre entier de 2 à 6.

5. Composition selon la revendication 1, dans laquelle le polymère de dépôt a une masse moléculaire moyenne en poids allant d'environ 3000 à environ 2 000 000.

6. Composition selon la revendication 1, dans laquelle le polymère de dépôt est anionique.

7. Composition selon la revendication 1, dans laquelle l'agent tensioactif cationique est un agent tensioactif cationique de sel de mono-alkyl ammonium quaternisé possédant un groupe alkyle ou alcényle long allant d'environ 12 à environ 30 atomes de carbone, ou sa combinaison avec un agent tensioactif cationique de sel de di-alkyl ammonium quaternisé possédant deux chaînes alkyle longues allant de 12 à 30 atomes de carbone.

8. Composition selon la revendication 1, dans laquelle l'agent tensioactif cationique est un agent tensioactif cationique de sel de mono-alkyl ammonium quaternisé possédant un groupe alkyle ou alcényle long de 22 atomes de carbone, ou sa combinaison avec un agent tensioactif cationique de sel de di-alkyl ammonium quaternisé possédant deux chaînes alkyle longues allant de 12 à 30 atomes de carbone.

9. Composition selon la revendication 1, dans laquelle le % molaire de l'agent tensioactif cationique par rapport à une somme de l'agent tensioactif cationique et du composé gras à point de fusion élevé va d'environ 22 % à environ 50 %.

10. Composition selon la revendication 1, dans laquelle le % molaire de l'agent tensioactif cationique par rapport à une somme de l'agent tensioactif cationique et du composé gras à point de fusion élevé va d'environ 25 % à environ 35 %.
